(19)

Europäisches
Patentamt
European
Patent Office
Office européen
des brevets

(11)  **EP 3 251 738 A1**

(12)  # EUROPEAN PATENT APPLICATION
published in accordance with Art. 153(4) EPC

(43) Date of publication:
**06.12.2017 Bulletin 2017/49**

(21) Application number: **16742981.0**

(22) Date of filing: **26.01.2016**

(51) Int Cl.:
**B01D 71/02** (2006.01)    **B01D 69/10** (2006.01)
**B01D 69/12** (2006.01)    **C01B 39/36** (2006.01)

(86) International application number:
**PCT/JP2016/000375**

(87) International publication number:
**WO 2016/121377 (04.08.2016 Gazette 2016/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **27.01.2015 JP 2015013666**

(71) Applicant: **Zeon Corporation
Tokyo 100-8246 (JP)**

(72) Inventors:
• **OMORI, Shiori
  Tokyo 100-8246 (JP)**
• **SUZUKI, Takahiro
  Tokyo 100-8246 (JP)**

(74) Representative: **Maiwald Patentanwalts GmbH
Elisenhof
Elisenstrasse 3
80335 München (DE)**

(54)  **SEPARATION MEMBRANE AND METHOD FOR PRODUCING SAME**

(57)    Provided is a separation membrane that when used in membrane separation of a mixture of a linear hydrocarbon and a branched hydrocarbon and/or cyclic hydrocarbon of equivalent carbon number to the linear hydrocarbon, can efficiently separate the linear hydrocarbon and the branched hydrocarbon and/or cyclic hydrocarbon. The separation membrane includes a porous support and a porous separation layer disposed on the porous support and containing an MFI-type zeolite. In an X-ray diffraction pattern obtained through X-ray diffraction measurement of the porous separation layer, the intensities of diffraction peaks attributed to specific MFI-type zeolite crystal planes satisfy specific relationships.

**EP 3 251 738 A1**

**Description**

TECHNICAL FIELD

This disclosure relates to a separation membrane and a method of producing the same, and in particular relates to a separation membrane that is suitable for use in separating one or more hydrocarbons from a hydrocarbon mixture and to a method of producing this separation membrane.

BACKGROUND

Membrane separation is conventionally used as a low-energy method for separating a branched hydrocarbon from a hydrocarbon mixture containing linear and branched hydrocarbons of equivalent carbon number. The separation membrane that is used may, for example, be a zeolite membrane that is obtained by forming a zeolite on a support in the form of a membrane.

In one specific example, PTL 1 discloses the separation of isobutene from a mixture of 1-butene and isobutene using a separation membrane that is a composite membrane (zeolite membrane) including a porous substrate and a porous separation layer that contains an MFI-type zeolite. Moreover, the zeolite membrane for separating isobutene in PTL 1 is produced by a method in which seed particles formed from silicate crystals of 30 nm to 100 nm in diameter are adhered to a porous substrate having a pore diameter of 1 nm to 5 nm, and then a zeolite is synthesized by hydrothermal synthesis on the porous substrate having the seed particles adhered thereto to form a porous separation layer.

CITATION LIST

Patent Literature

PTL 1: JP 2007-517648 A

SUMMARY

(Technical Problem)

However, there is room for improvement over the conventional zeolite membrane described above in terms that when the conventional zeolite membrane is used in membrane separation of a hydrocarbon mixture of a linear hydrocarbon and a branched hydrocarbon and/or cyclic hydrocarbon of equivalent carbon number to the linear hydrocarbon, the separation efficiency is inadequate.

Accordingly, an objective of this disclosure is to provide a separation membrane that when used in membrane separation of a hydrocarbon mixture of a linear hydrocarbon and a branched hydrocarbon and/or cyclic hydrocarbon of equivalent carbon number to the linear hydrocarbon, can efficiently separate the linear hydrocarbon and the branched hydrocarbon and/or cyclic hydrocarbon.

(Solution to Problem)

The inventors conducted diligent investigation to achieve the objective set forth above. The inventors discovered that a zeolite membrane having specific properties displays excellent separation efficiency when used in membrane separation of a hydrocarbon mixture of a linear hydrocarbon and a branched hydrocarbon and/or cyclic hydrocarbon of equivalent carbon number to the linear hydrocarbon. Moreover, the inventors discovered that this zeolite membrane having specific properties can be easily formed by adopting a combination of zeolite seed crystals having a specific average particle diameter and a porous support having a specific average pore diameter in formation of a zeolite membrane through hydrothermal synthesis of a zeolite including an MFI-type zeolite on a porous support having zeolite seed crystals adhered thereto. The inventors completed the disclosed techniques based on the new findings set forth above.

Specifically, the present disclosure aims to advantageously solve the problems set forth above by disclosing a separation membrane for use in membrane separation of a hydrocarbon mixture containing a linear hydrocarbon and either or both of a branched hydrocarbon and a cyclic hydrocarbon of equivalent carbon number to the linear hydrocarbon, the separation membrane comprising: a porous support; and a porous separation layer disposed on the porous support and containing an MFI-type zeolite, wherein in an X-ray diffraction pattern obtained through X-ray diffraction measurement of the porous separation layer: a total intensity of diffraction peaks attributed to MFI-type zeolite (001) planes, (002) planes, (004) planes, (101) planes, (102) planes, (103) planes, (104) planes, (105) planes, (202) planes, and (303) planes is at least 3 times the sum of a total intensity of diffraction peaks attributed to MFI-type zeolite (100) planes, (200) planes, (400) planes, (301) planes, and (501) planes and a total intensity of diffraction peaks attributed to MFI-type zeolite (010) planes, (020) planes, (040) planes, and (051) planes; and a total intensity of diffraction peaks attributed to MFI-type zeolite (101) planes, (102) planes, (103) planes, (104) planes, and (105) planes is less than 3 times an intensity of a diffraction peak attributed to MFI-type zeolite (101) planes. Through the separation membrane described above in which the porous separation layer disposed on the porous support contains an MFI-type zeolite and has an X-ray diffraction pattern in which the intensities of prescribed diffraction peaks satisfy specific relationships, a linear hydrocarbon and a branched hydrocarbon and/or cyclic hydrocarbon of equivalent carbon number to the linear hydrocarbon can be efficiently separated. In particular, this separation membrane enables highly efficient separation of a linear hydrocarbon and a branched hydrocarbon of equivalent carbon number.

The hydrocarbon mixture is preferably a mixture containing, as main components, a linear hydrocarbon having a carbon number of 4 and either or both of a branched hydrocarbon having a carbon number of 4 and a cyclic hydrocarbon having a carbon number of 4, or a mixture containing, as main components, a linear hydrocarbon having a carbon number of 5 and either or both of a branched hydrocarbon having a carbon number of 5 and a cyclic hydrocarbon having a carbon number of 5.

Herein, the term "zeolite" is inclusive of aluminosilicates such as ZSM-5 and silicalites. Moreover, the "X-ray diffraction measurement" referred to herein is performed by an X-ray diffractometer under conditions of a tube voltage of 30 kV and a tube current of 15 mA.

Furthermore, the present disclosure aims to advantageously solve the problems set forth above by disclosing a method of producing a separation membrane, for use in producing the above-described separation membrane, comprising immersing a porous support having one or more zeolite seed crystals adhered thereto in an aqueous sol containing a silica source and a structure directing agent, and synthesizing a zeolite including an MFI-type zeolite by hydrothermal synthesis to form a porous separation layer on the porous support, wherein the zeolite seed crystals have an average particle diameter of at least 50 nm and no greater than 700 nm, and a ratio of the average particle diameter of the zeolite seed crystals relative to an average pore diameter of the porous support is at least 0.01 and no greater than 0.7. Through use of zeolite seed crystals having an average particle diameter of at least 50 nm and no greater than 700 nm and by setting the ratio of the average particle diameter of the zeolite seed crystals relative to the average pore diameter of the porous support (average particle diameter/average pore diameter) as at least 0.01 and no greater than 0.7 as described above, it is easy to form a separation membrane in which a porous separation layer disposed on a porous support contains an MFI-type zeolite and has an X-ray diffraction pattern in which the intensities of prescribed diffraction peaks satisfy specific relationships.

The "average particle diameter of the zeolite seed crystals" referred to herein can be determined by calculating the number average of particle diameters of 20 zeolite seed crystals measured using a scanning electron microscope (SEM). Moreover, the "average pore diameter of the porous support" referred to herein can be determined by mercury intrusion porosimetry using a mercury porosimeter.

(Advantageous Effect)

According to this disclosure, it is possible to provide a separation membrane that when used in membrane separation of a hydrocarbon mixture of a linear hydrocarbon and a branched hydrocarbon and/or cyclic hydrocarbon of equivalent carbon number to the linear hydrocarbon, can efficiently separate the linear hydrocarbon and the branched hydrocarbon and/or cyclic hydrocarbon.

BRIEF DESCRIPTION OF THE DRAWING

In the accompanying drawing,

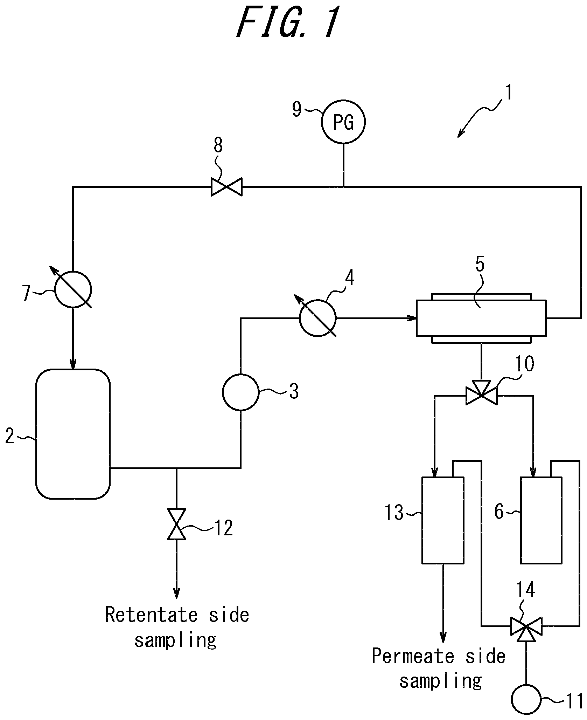

FIG. 1 illustrates an overview of configuration of a test apparatus used in the examples.

DETAILED DESCRIPTION

The following provides a detailed description of a disclosed embodiment.

The presently disclosed separation membrane can be used in membrane separation of a hydrocarbon mixture containing a linear hydrocarbon and a branched hydrocarbon and/or cyclic hydrocarbon (i.e., either or both of a branched hydrocarbon and a cyclic hydrocarbon) of equivalent carbon number to the linear hydrocarbon. The presently disclosed separation membrane can be produced, for example, using the presently disclosed method of producing a separation membrane.

(Separation membrane)

The presently disclosed separation membrane used in membrane separation of a hydrocarbon mixture is a so-called "zeolite membrane". The presently disclosed separation membrane includes a porous support and a porous separation layer disposed on the porous support. The porous separation layer contains an MFI-type zeolite (aluminosilicate and/or silicalite having an MFI structure). Moreover, an X-ray diffraction pattern obtained through X-ray diffraction measurement of the porous separation layer in the presently disclosed separation membrane satisfies the following conditions (1) and (2).

(1) The total intensity of diffraction peaks attributed to MFI-type zeolite (001) planes, (002) planes, (004) planes, (101) planes, (102) planes, (103) planes, (104) planes, (105) planes, (202) planes, and (303) planes is at least 3 times the sum of the total intensity of diffraction peaks attributed to MFI-type zeolite (100) planes, (200) planes, (400) planes, (301) planes, and (501) planes and the total intensity of diffraction peaks attributed to MFI-type zeolite (010) planes, (020) planes, (040) planes, and (051) planes.
(2) The total intensity of diffraction peaks attributed to MFI-type zeolite (101) planes, (102) planes, (103) planes, (104) planes, and (105) planes is less than 3 times the intensity of a diffraction peak attributed to MFI-type zeolite (101) planes.

As a result of the presently disclosed separation membrane including a porous separation layer that contains an MFI-type zeolite and that has an X-ray diffraction pattern satisfying conditions (1) and (2), when the presently disclosed separation membrane is used in membrane separation of a hydrocarbon mixture of a linear hydrocarbon and a branched hydrocarbon and/or cyclic hydrocarbon of equivalent carbon number to the linear hydrocarbon, the presently disclosed separation membrane can efficiently separate the linear hydrocarbon and the branched hydrocarbon and/or cyclic hydrocarbon. In particular, the presently disclosed separation membrane enables highly efficient separation of a linear hydrocarbon and a branched hydrocarbon.

<Hydrocarbon mixture>

The hydrocarbon mixture that is membrane separated using the presently disclosed separation membrane is a mixture that contains a linear hydrocarbon and a branched hydrocarbon and/or cyclic hydrocarbon of equivalent carbon number to the linear hydrocarbon. Moreover, this hydrocarbon mixture is preferably a mixture that contains, as main components, a linear hydrocarbon having a carbon number of 4 and a branched hydrocarbon having a carbon number of 4 and/or a cyclic hydrocarbon having a carbon number of 4, or a mixture that contains, as main components, a linear hydrocarbon having a carbon number of 5 and a branched hydrocarbon having a carbon number of 5 and/or a cyclic hydrocarbon having a carbon number of 5. Furthermore, this hydrocarbon mixture is more preferably a mixture that contains, as main components, a linear hydrocarbon having a carbon number of 5 and a branched hydrocarbon having a carbon number of 5 and/or a cyclic hydrocarbon having a carbon number of 5. The presently disclosed separation membrane enables efficient separation of a hydrocarbon mixture containing, as main components, a linear hydrocarbon having a carbon number of 4 or 5 and a branched hydrocarbon and/or cyclic hydrocarbon of equivalent carbon number to the linear hydrocarbon. In particular, the presently disclosed separation membrane enables efficient separation of a hydrocarbon mixture containing, as main components, a linear hydrocarbon having a carbon number of 5 and a branched hydrocarbon having a carbon number of 5 and/or a cyclic hydrocarbon having a carbon number of 5.
Herein, the phrase "containing, as main components, a linear hydrocarbon and a branched hydrocarbon and/or cyclic hydrocarbon" means that the hydrocarbon mixture comprises at least 50 mol%, in total, of the linear hydrocarbon and the branched hydrocarbon and/or cyclic hydrocarbon.
The mixture containing, as main components, a linear hydrocarbon having a carbon number of 4 and a branched hydrocarbon and/or cyclic hydrocarbon having a carbon number of 4 may, for example, be a mixture containing a linear hydrocarbon having a carbon number of 4 such as n-butane, 1-butene, 2-butene, or butadiene, and a branched hydrocarbon having a carbon number of 4 such as isobutane or isobutene and/or a cyclic hydrocarbon having a carbon number of 4 such as cyclobutane or cyclobutene. Specifically, the mixture containing, as main components, a linear hydrocarbon having a carbon number of 4 and a branched hydrocarbon and/or cyclic hydrocarbon having a carbon number of 4 may, for example, be a C4 fraction obtained as a by-product in thermal cracking of naphtha to produce ethylene or a fraction that remains after removing at least some butadiene from this C4 fraction.
The mixture containing, as main components, a linear hydrocarbon having a carbon number of 5 and a branched hydrocarbon and/or cyclic hydrocarbon having a carbon number of 5 may, for example, be a mixture containing a linear hydrocarbon having a carbon number of 5 such as n-pentane, 1-pentene, 2-pentene, or 1,3-pentadiene, and a branched hydrocarbon having a carbon number of 5 such as isopentane, 2-methyl-1-butene, 2-methyl-2-butene, 3-methyl-1-butene, or isoprene and/or a cyclic hydrocarbon having a carbon number of 5 such as cyclopentane or cyclopentene.

Specifically, the mixture containing, as main components, a linear hydrocarbon having a carbon number of 5 and a branched hydrocarbon and/or cyclic hydrocarbon having a carbon number of 5 may, for example, be a C5 fraction obtained as a by-product in thermal cracking of naphtha to produce ethylene or a fraction that remains after removing at least some isoprene from this C5 fraction.

<Porous support>

The porous support is a porous body having pores therein. The porous support can be a porous body made of any material so long as the porous support is a porous body capable of supporting the porous separation layer. Of such porous bodies, a porous body made from a porous ceramic such as alumina, mullite, zirconia, or cordierite or a porous sintered metal such as stainless steel is preferable. This is because a porous body made of a porous ceramic or a porous sintered metal has excellent mechanical strength.

The porous support may have any shape such as a flat film shape, a flat plate shape, a tube shape, or a honeycomb shape without any specific limitations.

The average pore diameter of the porous support is preferably at least 0.1 $\mu$m, more preferably at least 0.5 $\mu$m, even more preferably at least 0.7 $\mu$m, and particularly preferably at least 1.0 $\mu$m, and is preferably no greater than 10 $\mu$m, more preferably no greater than 5.0 $\mu$m, even more preferably no greater than 3.0 $\mu$m, and particularly preferably no greater than 2.0 $\mu$m.

<Porous separation layer>

The porous separation layer can be formed by, for example, synthesizing a zeolite including an MFI-type zeolite on a porous support having one or more zeolite seed crystals adhered thereto. From a viewpoint of adequately raising separation efficiency with respect to a linear hydrocarbon and a branched hydrocarbon and/or cyclic hydrocarbon of equivalent carbon number to the linear hydrocarbon, the porous separation layer of the presently disclosed separation membrane is required to contain an MFI-type zeolite and have an X-ray diffraction pattern satisfying specific conditions. In particular, it is preferable that the porous separation layer in the presently disclosed separation membrane is substantially formed from an MFI-type zeolite.

[X-ray diffraction pattern]

The porous separation layer is specifically required to have an X-ray diffraction pattern in which the total intensity of diffraction peaks attributed to MFI-type zeolite (001) planes, (002) planes, (004) planes, (101) planes, (102) planes, (103) planes, (104) planes, (105) planes, (202) planes, and (303) planes (hereinafter, also referred to as "peak intensity attributed to a c-axis") is at least 3 times the sum of the total intensity of diffraction peaks attributed to MFI-type zeolite (100) planes, (200) planes, (400) planes, (301) planes, and (501) planes (hereinafter, also referred to as "peak intensity attributed to an a-axis") and the total intensity of diffraction peaks attributed to MFI-type zeolite (010) planes, (020) planes, (040) planes, and (051) planes (hereinafter, also referred to as "peak intensity attributed to a b-axis").

From a viewpoint of further improving separation efficiency with respect to a linear hydrocarbon and a branched hydrocarbon and/or cyclic hydrocarbon of equivalent carbon number to the linear hydrocarbon, the peak intensity attributed to the c-axis in the X-ray diffraction pattern of the porous separation layer is preferably at least 4 times, more preferably at least 5 times, even more preferably at least 5.3 times, and particularly preferably at least 5.5 times the sum of the peak intensity attributed to the a-axis and the peak intensity attributed to the b-axis, and is preferably no greater than 7 times, more preferably no greater than 6.3 times, and even more preferably no greater than 6 times the sum of the peak intensity attributed to the a-axis and the peak intensity attributed to the b-axis.

Moreover, the porous separation layer is required to have an X-ray diffraction pattern in which the total intensity of diffraction peaks attributed to MFI-type zeolite (101) planes, (102) planes, (103) planes, (104) planes, and (105) planes is less than 3 times the intensity of a diffraction peak attributed to MFI-type zeolite (101) planes.

From a viewpoint of further improving separation efficiency with respect to a linear hydrocarbon and a branched hydrocarbon and/or cyclic hydrocarbon of equivalent carbon number to the linear hydrocarbon, the total intensity of diffraction peaks attributed to MFI-type zeolite (101) planes, (102) planes, (103) planes, (104) planes, and (105) planes in the X-ray diffraction pattern of the porous separation layer is preferably greater than the intensity of the diffraction peak attributed to MFI-type zeolite (101) planes, more preferably at least 1.1 times, even more preferably at least 1.2 times, and particularly preferably at least 1.4 times the intensity of the diffraction peak attributed to MFI-type zeolite (101) planes, and is preferably no greater than 2.7 times, more preferably no greater than 2.5 times, even more preferably no greater than 2.3 times, and particularly preferably no greater than 2.0 times the intensity of the diffraction peak attributed to MFI-type zeolite (101) planes.

The intensities of diffraction peaks in the X-ray diffraction pattern of the porous separation layer can be adjusted by, for example, altering separation membrane production conditions such as the properties of the porous support, the properties of the zeolite seed crystals adhered to the porous support, and the method by which the zeolite seed crystals are adhered to the porous support, but the method of adjustment is not specifically limited.

[Layer thickness]

The thickness of the porous separation layer is preferably at least 1 $\mu$m, more preferably at least 3 $\mu$m, even more preferably at least 5 $\mu$m, and particularly preferably at least 7 $\mu$m, and is preferably no greater than 50 $\mu$m, more preferably no greater than 40 $\mu$m, even more preferably no greater than 30 $\mu$m, and particularly preferably no greater than 15 $\mu$m. Setting the thickness of the porous separation layer as at least 1 $\mu$m can inhibit the formation of pin holes and can raise the separation factor of the separation membrane, which further improves separation efficiency with respect to a linear hydrocarbon and a branched hydrocarbon and/or cyclic hydrocarbon of equivalent carbon number to the linear hydrocarbon. Moreover, setting the thickness of the porous separation layer as no greater than 50 $\mu$m can suppress a decrease in permeation flux of the separation membrane, and thereby further improve separation efficiency with respect to a linear hydrocarbon and a branched hydrocarbon and/or cyclic hydrocarbon of equivalent carbon number to the linear hydrocarbon.

The thickness of the porous separation layer can be measured using a scanning electron microscope (SEM). Moreover, the thickness of the porous separation layer can be controlled by adjusting the average particle diameter of zeolite seed crystals used to form the porous separation layer, the zeolite synthesis conditions (for example, temperature and time), and so forth.

(Method of producing separation membrane)

The presently disclosed separation membrane including the porous separation layer having the properties set forth above can, for example, be easily produced by the presently disclosed method of producing a separation membrane.

The presently disclosed method of producing a separation membrane includes immersing a porous support having one or more zeolite seed crystals adhered thereto in an aqueous sol containing a silica source and a structure directing agent, and synthesizing a zeolite including an MFI-type zeolite by hydrothermal synthesis to form a porous separation layer on the porous support (separation layer formation step), and may optionally further include preparing the zeolite seed crystals (seed crystal preparation step) and adhering the zeolite seed crystals prepared in the seed crystal preparation step to the porous support (seed crystal adhesion step).

In the presently disclosed method of producing a separation membrane, it is a requirement to use zeolite seed crystals having an average particle diameter of at least 50 nm and no greater than 700 nm and to use a combination of a porous support and zeolite seed crystals that enable a ratio of the average particle diameter of the zeolite seed crystals relative to the average pore diameter of the porous support of at least 0.01 and no greater than 0.7. In other words, the zeolite seed crystals used in the presently disclosed method of producing a separation membrane are required to have a specific average particle diameter that is smaller than the average pore diameter of the porous support.

Although it is not clear why a porous separation layer having the properties set forth above can be easily formed by using zeolite seed crystals having an average particle diameter of at least 50 nm and no greater than 700 nm and using a combination of a porous support and zeolite seed crystals that enable a ratio of the average particle diameter of the zeolite seed crystals relative to the average pore diameter of the porous support of at least 0.01 and no greater than 0.7, the reason for this is presumed to be as follows. Specifically, it is presumed that when zeolite seed crystals having the average particle diameter set forth above and a porous support having the average pore diameter set forth above are used, the zeolite seed crystals enter the pores of the porous support, which suitably restricts the direction of zeolite growth, and thus a porous separation layer having the properties set forth above can be easily formed.

From a viewpoint of forming a porous separation layer having favorable properties and obtaining a separation membrane having excellent separation efficiency, the average particle diameter of the zeolite seed crystals is preferably at least 100 nm, more preferably at least 150 nm, even more preferably at least 200 nm, and particularly preferably at least 300 nm, and is preferably no greater than 600 nm, more preferably no greater than 500 nm, even more preferably no greater than 400 nm, and particularly preferably no greater than 350 nm.

Moreover, from a viewpoint of forming a porous separation layer having favorable properties and obtaining a separation membrane having excellent separation efficiency, the ratio of the average particle diameter of the zeolite seed crystals relative to the average pore diameter of the porous support is preferably at least 0.05, more preferably at least 0.1, even more preferably at least 0.15, and particularly preferably at least 0.2, and is preferably no greater than 0.5, more preferably no greater than 0.4, and even more preferably no greater than 0.3.

In the presently disclosed method of producing a separation membrane, the porous support having zeolite seed crystals adhered thereto may be obtained by synthesizing a zeolite that then serves as seed crystals on a porous support that does not already have zeolite seed crystals adhered thereto. However, from a viewpoint of forming a porous separation layer having favorable properties and obtaining a separation membrane having excellent separation efficiency, the porous support having zeolite seed crystals adhered thereto is preferably obtained by adhering zeolite seed crystals that have been prepared in advance onto a porous support. In other words, the presently disclosed method of producing a separation membrane preferably further includes a seed crystal preparation step and a seed crystal adhesion step.

<Seed crystal preparation step>

The seed crystal preparation step may be a step in which zeolite seed crystals having an average particle diameter of at least 50 nm and no greater than 700 nm are produced by a known method of producing zeolite seed crystals, but is not specifically limited thereto. The zeolite seed crystals preferably include an MFI-type zeolite, and are more preferably substantially formed from an MFI-type zeolite.

Specifically, the seed crystal preparation step may involve, for example, preparing zeolite seed crystals having an average particle diameter of at least 50 nm and no greater than 700 nm by heating an aqueous sol for seed crystals obtained through mixing of a silica source, a structure directing agent, and water, producing coarse zeolite crystals by hydrothermal synthesis, and then optionally drying and grinding the coarse crystals that are obtained.

[Aqueous sol for seed crystals]

Examples of silica sources that can be used in preparation of the zeolite seed crystals include, but are not specifically limited to, colloidal silica, wet silica, amorphous silica, fumed silica, sodium silicate, silica sol, silica gel, kaolinite, diatomite, aluminum silicate, white carbon black, tetrabutoxysilane, tetrabutyl orthosilicate, and tetraethoxysilane. Of these silica sources, tetraethoxysilane and colloidal silica are preferable, and tetraethoxysilane is more preferable.

Examples of structure directing agents that can be used include, but are not specifically limited to, alcohols and quaternary ammonium salts such as tetraethylammonium hydroxide, tetrapropylammonium hydroxide, and tetrapropylammonium bromide. Of these structure directing agents, tetraethylammonium hydroxide, tetrapropylammonium hydroxide, and tetrapropylammonium bromide are preferable. Although the mixing ratio of the structure directing agent is not specifically limited, a molar ratio of the silica source and the structure directing agent (silica source:structure directing agent) is preferably in a range of 1:0.01 to 1:2.0, more preferably in a range of 1:0.1 to 1:1.0, and even more preferably in a range of 1:0.15 to 1:0.8.

Moreover, although the mixing ratio of water in the aqueous sol for seed crystals is not specifically limited, a molar ratio of the silica source and water (silica source:water) is preferably in a range of 1:3 to 1:100, and more preferably in a range of 1:5 to 1:50.

[Hydrothermal synthesis of coarse crystals]

The heating temperature when the aqueous sol for seed crystals is heated to obtain coarse crystals by hydrothermal synthesis is preferably at least 100°C and no higher than 200°C, and more preferably at least 130°C and no higher than 150°C. The heating time is preferably at least 10 hours and no greater than 50 hours, and more preferably at least 20 hours and no greater than 50 hours.

The hydrothermal synthesis is typically carried out by adding the aqueous sol for seed crystals into a pressure vessel and then heating the pressure vessel under the conditions set forth above. Examples of pressure vessels that can be used include, but are not specifically limited to, a stainless steel pressure vessel including a fluororesin inner cylinder, a nickel metal pressure vessel, and a fluororesin pressure vessel. Examples of methods by which the pressure vessel can be heated include a method in which the pressure vessel is heated in a hot-air dryer and a method in which the pressure vessel is heated by a directly attached heater.

The coarse crystals obtained through heating of the aqueous sol for seed crystals can be collected by a known solid-liquid separation technique such as centrifugal separation. The coarse crystals that are collected may be used as zeolite seed crystals as-collected, or may be used as zeolite seed crystals after drying and grinding.

[Drying and grinding of coarse crystals]

The temperature at which the collected coarse crystals are dried is preferably at least 70°C and no higher than 100°C, but is not specifically limited to this range. Moreover, no specific limitations are placed on the grinding method and conditions in grinding of the coarse crystals, and a method and conditions that enable a desired average particle diameter to be obtained are adopted.

<Seed crystal adhesion step>

In the seed crystal adhesion step, the zeolite seed crystals may be adhered to (mounted on) the porous support by a known technique such as coating or rubbing. Specifically, zeolite seed crystals may be adhered to a porous support in the seed crystal adhesion step by applying, onto the porous support, a dispersion liquid obtained by dispersing zeolite seed crystals having an average particle diameter of at least 50 nm and no greater than 700 nm in water, and then drying the dispersion liquid that has been applied. Alternatively, zeolite seed crystals may be adhered to a porous support in the seed crystal adhesion step by rubbing zeolite seed crystals having an average particle diameter of at least 50 nm and no greater than 700 nm onto a porous support that has been wetted in advance through immersion in ultrapure water for 1 minute to 60 minutes. From a viewpoint of adhering zeolite seed crystals to a porous support in a high density, zeolite seed crystals are preferably adhered to a porous support in the seed crystal adhesion step by rubbing the zeolite seed crystals onto a pre-wetted porous support.

The porous support to which the zeolite seed crystals are adhered may be any porous support that enables a ratio of the average particle diameter of the zeolite seed crystals relative to the average pore diameter of the porous support of at least 0.01 and no greater than 0.7. Specifically, the porous support may be any of the porous supports described in the preceding "<Porous support>" section.

The adhered zeolite seed crystals can be fixed to the porous support by removing moisture contained in the porous support by drying. The temperature during this drying is preferably at least 70°C and no higher than 100°C, but is not specifically limited to this range.

<Separation layer formation step>

In the separation layer formation step, the porous support having the zeolite seed crystals adhered thereto is immersed in an aqueous sol containing a silica source and a structure directing agent, and a zeolite including an MFI-type zeolite is synthesized by hydrothermal synthesis to form a porous separation layer on the porous support. A separation membrane obtained by forming the porous separation layer on the porous support in the separation layer formation step may be optionally subjected to boil washing and/or firing treatment.

[Aqueous sol]

The aqueous sol used in formation of the porous separation layer can be prepared by mixing a silica source, a structure directing agent, and water.

Examples of silica sources that can be used include, but are not specifically limited to, colloidal silica, wet silica, amorphous silica, fumed silica, sodium silicate, silica sol, silica gel, kaolinite, diatomite, aluminum silicate, white carbon black, tetrabutoxysilane, tetrabutyl orthosilicate, and tetraethoxysilane. Of these silica sources, tetraethoxysilane and colloidal silica are preferable, and tetraethoxysilane is more preferable.

Examples of structure directing agents that can be used include, but are not specifically limited to, crown ethers, alcohols, and quaternary ammonium salts such as tetraethylammonium hydroxide, tetrapropylammonium hydroxide, and tetrapropylammonium bromide. Of these structure directing agents, tetraethylammonium hydroxide, tetrapropylammonium hydroxide, and tetrapropylammonium bromide are preferable, and a combination of tetrapropylammonium hydroxide and tetrapropylammonium bromide is more preferable.

Although the mixing ratio of the structure directing agent in the aqueous sol is not specifically limited, a molar ratio of the silica source and the structure directing agent (silica source:structure directing agent) is preferably in a range of 1:0.01 to 1:2.0, more preferably in a range of 1:0.1 to 1:1.0, and even more preferably in a range of 1:0.15 to 1:0.8.

Moreover, although the mixing ratio of water in the aqueous sol is not specifically limited, a molar ratio of the silica source and water (silica source:water) is preferably in a range of 1:100 to 1:1000, and more preferably in a range of 1:200 to 1:800.

[Zeolite hydrothermal synthesis]

The method by which the porous support having the zeolite seed crystals adhered thereto is immersed in the aqueous sol may be, but is not specifically limited to, a method in which the aqueous sol is added into a pressure vessel housing the porous support having the zeolite seed crystals adhered thereto. Alternatively, a method may be adopted in which the porous support having the zeolite seed crystals adhered thereto is placed in a pressure vessel containing the aqueous sol. The pressure vessel used for this immersion may be the same as any of the pressure vessels that can be used in preparation of the zeolite seed crystals.

In heating of the aqueous sol in which the porous support having the zeolite seed crystals adhered thereto is immersed and synthesis of a zeolite including an MFI-type zeolite by hydrothermal synthesis to form the porous separation layer on the porous support, the heating temperature is preferably at least 100°C and no higher than 250°C, and more preferably at least 150°C and no higher than 200°C. The heating time is preferably at least 1 hour and no greater than 50 hours, and more preferably at least 2 hours and no greater than 20 hours. Examples of methods by which the aqueous sol and the porous support in the pressure vessel can be heated include a method in which the pressure vessel is heated in a hot-air dryer and a method in which the pressure vessel is heated by a directly attached heater.

[Boil washing]

A washing liquid used in boil washing of the separation membrane obtained through formation of the porous separation layer on the porous support may, for example, be distilled water. The boil washing time is preferably at least 10 minutes and no greater than 2 hours, and more preferably at least 30 minutes and no greater than 1.5 hours. Note that the boil washing may be repeated (for example, 2 or 3 times) and that the repetitions of the boil washing may each be carried out under the same boil washing conditions or different boil washing conditions. Moreover, drying treatment may be performed after the boil washing as necessary. The drying temperature of the separation membrane after the boil washing is preferably at least 70°C and no higher than 100°C.

[Firing treatment]

The separation membrane obtained through formation of the porous separation layer on the porous support is preferably subjected to firing treatment to remove the structure directing agent. The heating rate in the firing treatment is preferably at least 0.1°C/minute and no greater than 1°C/minute, and more preferably at least 0.1°C/minute and no greater than 0.5°C/minute. The firing temperature is preferably at least 400°C and no higher than 800°C, and more preferably at least 400°C and no higher than 600°C. Moreover, the cooling rate is preferably at least 0.1 °C/minute and no greater than 1°C/minute, and more preferably at least 0.1°C/minute and no greater than 0.4°C/minute. The firing time (hold time) is preferably at least 1 hour and no greater than 30 hours, and more preferably at least 5 hours and no greater than 30 hours.

(Method of hydrocarbon mixture membrane separation)

In membrane separation of a hydrocarbon mixture using the presently disclosed separation membrane produced by the presently disclosed method of producing a separation membrane, a linear hydrocarbon, for example, can be efficiently separated and removed from a hydrocarbon mixture containing the linear hydrocarbon and a branched hydrocarbon and/or cyclic hydrocarbon of equivalent carbon number to the linear hydrocarbon, and, as a result, the percentage content of the branched hydrocarbon and/or cyclic hydrocarbon in the hydrocarbon mixture can be increased. Specifically, in membrane separation of a hydrocarbon mixture using the presently disclosed separation membrane, one or more components (for example, a linear hydrocarbon) can be separated and removed from the hydrocarbon mixture by passing the hydrocarbon mixture through the separation membrane.

The membrane separation is preferably carried out under heated conditions. Specifically, the membrane separation is carried out under conditions of preferably at least 20°C and no higher than 300°C, more preferably at least 25°C and no higher than 250°C, and even more preferably at least 50°C and no higher than 200°C. Although no specific limitations are placed on pressure conditions during the membrane separation, a pressure difference between a retentate side and a permeate side of the separation membrane (pressure at retentate side - pressure at permeate side) is preferably at least 10 kPa and no greater than 600 kPa, and more preferably at least 50 kPa and no greater than 300 kPa.

EXAMPLES

The following provides a more specific description of the present disclosure based on examples. However, the present disclosure is not limited to the following examples. In the following description, "%" and the like used to express quantities are by mass, unless otherwise specified.

In the examples and comparative examples, the following methods were used to measure and evaluate the average particle diameter of zeolite seed crystals, the average pore diameter of a porous support, the thickness of a porous separation layer, the X-ray diffraction pattern of a porous separation layer, and the performance of a separation membrane.

<Average particle diameter of zeolite seed crystals>

The particle diameters of 20 zeolite seed crystals were measured using a scanning electron microscope (SEM). The average value of the measured values was calculated and was taken to be the average particle diameter of the zeolite seed crystals.

<Average pore diameter of porous support>

The average pore diameter of a porous support was determined by mercury intrusion porosimetry using a mercury porosimeter (PoreMaster 60GT produced by Quantachrome Instruments). In measurement by mercury intrusion porosimetry using the mercury porosimeter, the pore diameter was determined by modeling pores as cylindrical shapes and using the Washburn equation: $-4\sigma\cos\theta = PD$ (in the Washburn equation, $\sigma$ represents the surface tension (N/m) of mercury, $\theta$ represents the contact angle (deg), D represents the pore diameter (m), and P represents the pressure (Pa)).

<Thickness of porous separation layer>

The thickness of a porous separation layer formed on a porous support was measured using a scanning electron microscope (SEM).

<X-ray diffraction pattern of porous separation layer>

An X-ray diffraction pattern of a porous separation layer was obtained using an X-ray diffractometer (Discover D8 produced by Bruker AXS). The measurement conditions were as follows.

X-ray source: Cu-K$\alpha$ radiation
Wavelength $\lambda$: 1.54 Angstroms
Tube voltage: 30 kV
Tube current: 15 mA
Power: 0.9 kW
Incident slit: length 1.0 mm $\times$ width 1.0 mm
Receiving slit: Soller slit (angular resolution 0.35 deg)
Detector: scintillation counter
Measurement rate: 0.01 deg/s

<Performance of separation membrane>

The results of a membrane separation test were used to calculate permeation flux F according to the following equation (I). Moreover, the separation factor $\alpha$ was calculated according to the following equation (II-1) or the following equation (II-2). Specifically, the separation factor $\alpha$ was calculated according to equation (II-1) in a situation in which a hydrocarbon mixture formed from a mixed liquid of n-pentane and isopentane was used as a feedstock and was calculated according to equation (II-2) when a hydrocarbon mixture formed from a mixture of n-pentane and cyclopentane was used as a feedstock. Moreover, separation efficiency was evaluated by calculating F $\times$ $\alpha$. A larger value for F $\times$ $\alpha$ indicates better separation efficiency.

$$F = W/(A \times t) \qquad\qquad (I)$$

$$\alpha = (Y_n/Y_{iso})/(X_n/X_{iso}) \qquad\qquad (II\text{-}1)$$

$$\alpha = (Y_n/Y_{cy})/(X_n/X_{cy}) \qquad\qquad (II\text{-}2)$$

In equation (I), W represents the mass [kg] of a component that has passed through the separation membrane, A represents the effective area [$m^2$] of the separation membrane, and t represents processing time [h]. In equation (II-1), $X_n$ represents the percentage content [mol%] of n-pentane in the feedstock, $X_{iso}$ represents the percentage content [mol%] of isopentane in the feedstock, $Y_n$ represents the percentage content [mol%] of n-pentane in a permeate side sample, and $Y_{iso}$ represents the percentage content [mol%] of isopentane in the permeate side sample. In equation (II-2), $X_n$ represents the percentage content [mol%] of n-pentane in the feedstock, $X_{cy}$ represents the percentage content [mol%] of cyclopentane in the feedstock, $Y_n$ represents the percentage content [mol%] of n-pentane in a permeate side sample, and $Y_{cy}$ represents the percentage content [mol%] of cyclopentane in the permeate side sample.

(Example 1)

<Preparation of aqueous sol A for seed crystals>

A magnetic stirrer was used to mix 152.15 g of a tetrapropylammonium hydroxide aqueous solution of 22.5 mass% in concentration (produced by Tokyo Chemical Industry Co., Ltd.; 34.23 g in terms of tetrapropylammonium hydroxide as structure directing agent) and 48.44 g of ultrapure water. In addition, 99.41 g of tetraethoxysilane (produced by Sigma-Aldrich Co. LLC.) was added as a silica source and mixing was performed for 70 minutes at room temperature using the magnetic stirrer to yield an aqueous sol A for seed crystal preparation.

<Preparation of zeolite seed crystals A>

The aqueous sol A for seed crystals was added into a stainless steel pressure vessel including a fluororesin inner cylinder, and then a reaction (hydrothermal synthesis) was carried out for 48 hours in a 130°C hot-air dryer. Next, solid-liquid separation of the resultant reaction liquid was performed for 5 minutes by centrifugal separation in a centrifugal separator (4000 rpm), and solid content was collected. The collected solid content was dried for 12 hours in an 80°C thermostatic dryer, and then the dried solid was ground in a mortar to yield zeolite seed crystals A. It was confirmed that the resultant zeolite seed crystals A were an MFI-type zeolite by X-ray diffraction measurement. The zeolite seed crystals A had an average particle diameter of 130 nm.

<Adhesion of zeolite seed crystals to porous support>

A circular tube-shaped porous support made of mullite (product name: PM Tube; produced by Nikkato Corporation; outer diameter: 12 mm; inner diameter 9 mm; length: 100 mm; average pore diameter: 1.4 $\mu$m; porosity: 42.7%) was washed with acetone, subsequently dried, and then immersed in ultrapure water for 10 minutes. After this immersion in ultrapure water, 0.05 g of the zeolite seed crystals A obtained as described above were rubbed onto the outer surface of the wet porous support and were dried for 12 hours in an 80°C dryer to adhere the zeolite seed crystals A to the surface of the porous support.

<Preparation of aqueous sol for porous separation layer>

A magnetic stirrer was used to mix 4.99 g of a tetrapropylammonium hydroxide aqueous solution of 22.5 mass% in concentration (produced by Tokyo Chemical Industry Co., Ltd.; 1.12 g in terms of tetrapropylammonium hydroxide as structure directing agent), 0.74 g of tetrapropylammonium bromide (produced by Wako Pure Chemical Industries, Ltd.) as a structure directing agent, and 238.79 g of ultrapure water for 10 minutes at room temperature. In addition, 6.71 g of tetraethoxysilane (produced by Sigma-Aldrich Co. LLC.) was added as a silica source and mixing was performed for 60 minutes at room temperature using the magnetic stirrer to yield an aqueous sol for porous separation layer formation. The composition of the aqueous sol, by molar ratio, was tetraethoxysilane: tetrapropylammonium hydroxide: tetrapropylammonium bromide:water = 1:0.2:0.1:419.

<Formation of porous separation layer>

The aqueous sol for a porous separation layer obtained as described above was added into a stainless steel pressure vessel. Next, the porous support having the zeolite seed crystals A adhered thereto was immersed in the aqueous sol for a porous separation layer, and a reaction (hydrothermal synthesis) was carried out for 14 hours in a 185°C hot-air dryer to form a porous separation layer on the porous support. The porous support having the porous separation layer formed thereon was subjected to two repetitions of boil washing for 1 hour using distilled water as a washing liquid. Thereafter, the porous support having the porous separation layer formed thereon was dried for 12 hours using an 80°C thermostatic dryer. Next, firing was performed to remove the structure directing agent (tetrapropylammonium hydroxide and tetrapropylammonium bromide) contained in the porous separation layer, and thereby obtain a separation membrane. The firing conditions were as follows.

    Heating rate: 0.25°C/minute
    Firing temperature: 500°C
    Firing time (hold time): 20 hours
    Cooling rate: 0.38°C/minute

The thickness of the porous separation layer in the resultant separation membrane was measured. Moreover, X-ray diffraction measurement of the porous separation layer was performed to obtain an X-ray diffraction pattern. As a result, it was confirmed that the porous separation layer was an MFI-type zeolite based on the obtained X-ray diffraction pattern. The intensities of diffraction peaks attributed to various MFI-type zeolite crystal planes were determined from the obtained X-ray diffraction pattern. Moreover, the magnitude of peak intensity attributed to a c-axis relative to the sum of peak intensity attributed to an a-axis and peak intensity attributed to a b-axis (hereinafter, also referred to as "c-axis/(a-axis + b-axis)") and the magnitude of intensities of diffraction peaks attributed to (101) planes, (102) planes, (103) planes, (104) planes, and (105) planes relative to the intensity of a diffraction peak attributed to (101) planes (hereinafter, also referred to as "Σ(10x)/(101)") were calculated. The results are shown in Table 1.

<Membrane separation test>

The separation membrane obtained as described above was subjected to a membrane separation test using a test apparatus 1 illustrated in FIG. 1.

[Test apparatus]

The test apparatus 1 illustrated in FIG. 1 includes a feedstock tank 2, a liquid feed pump 3, a first heat exchanger 4, a separator 5, and a second heat exchanger 7. The separator 5 is configured by setting up the separation membrane obtained as described above in a circular tube. The test apparatus 1 illustrated in FIG. 1 also includes a cold trap 6 and a sampling cold trap 13 that are connected to the separator 5 via a three-way valve 10, and a vacuum pump 11 that is connected downstream of the cold trap 6 and the cold trap 13 via a three-way valve 14. Moreover, the test apparatus 1 includes a sampling valve 12 between the feedstock tank 2 and the liquid feed pump 3, and a back pressure valve 8 and a pressure gauge 9 downstream of the separator 5.

In the test apparatus 1 illustrated in FIG. 1, a feedstock loaded into the feedstock tank 2 is fed to the first heat exchanger 4 by the liquid feed pump 3 and is heated to a temperature at least as high as the temperature at which the feedstock vaporizes. The vaporized feedstock is fed to the separator 5 as a gas phase and then undergoes separation (membrane separation) of components by the separator 5 including the separation membrane. In the test apparatus 1, the vacuum pump 11 is used to maintain a reduced pressure state at the permeate side of the separation membrane. A permeate that has passed through the separation membrane is fed to the connected cold trap 6 or sampling cold trap 13 via the three-way valve 10. On the other hand, a retentate that has not passed through the separation membrane in the separator 5 is condensed through cooling by the second heat exchanger 7 and is returned to the feedstock tank 2. Note that back pressure in the test apparatus 1 is adjusted by the back pressure valve 8 and the pressure gauge 9 provided downstream of the separator 5. In the test apparatus 1, a permeate that has passed through the separation membrane in the separator 5 can be extracted as a permeate side sample through switching of the three-way valves 10 and 14.

[Membrane separation]

The membrane separation test was implemented as follows using the test apparatus 1 illustrated in FIG. 1.

Specifically, a C5 hydrocarbon mixture formed from a mixed liquid of n-pentane and isopentane (mixed liquid comprising 50 mol% of n-pentane and 50 mol% of isopentane) was first loaded into the feedstock tank 2 and a degassing operation was performed three times. Thereafter, a feedstock circulation process was initiated in which the hydrocarbon mixture was fed to the separator 5 by the liquid feed pump 3, via the first heat exchanger 4 heated to 70°C so as to be fed as a gas phase, and was then condensed by the second heat exchanger 7 and returned to the feedstock tank 2. After the feedstock circulation process had been initiated, operation was continued until the temperature of the system reached a steady state. Once the temperature of the system reached a steady state, the back pressure valve 8 was used to increase the pressure at the retentate side to 50 kPa and the vacuum pump 11 was operated to reduce the pressure at the permeate side (cold trap 6 and cold trap 13) to -100 kPa. After a stable temperature and pressure had been confirmed in the system, the three-way valve 10 at the permeate side was opened to start the membrane separation test. In other words, the membrane separation test was performed at a temperature of 70°C and with a pressure difference between the retentate side and the permeate side of 150 kPa.

Extraction of a sample at the permeate side was started after 5 minutes had passed from the start of the membrane separation test. Specifically, the three-way valves 10 and 14 were used to switch the flow path at the permeate side from the cold trap 6 to the sampling cold trap 13, and a permeate side sample was captured and extracted in the sampling cold trap 13 as a condensate. The sampling time during this was set as 10 minutes. The permeate side sample (condensate) was weighed and was measured by gas chromatography to determine the molar ratio of n-pentane and isopentane. The performance of the separation membrane was evaluated using these measurement results. The results are shown in Table 1.

(Example 2)

A separation membrane was prepared and evaluated in the same way as in Example 1 with the exception that zeolite seed crystals B prepared as follows were used instead of the zeolite seed crystals A. The results are shown in Table 1. It was confirmed that the porous separation layer of the separation membrane was an MFI-type zeolite as a result of X-ray diffraction measurement of the porous separation layer.

<Preparation of aqueous sol B for seed crystals>

A magnetic stirrer was used to mix 69.23 g of a tetrapropylammonium hydroxide aqueous solution of 22.5 mass% in concentration (produced by Tokyo Chemical Industry Co., Ltd.; 15.58 g in terms of tetrapropylammonium hydroxide as structure directing agent) and 165.64 g of ultrapure water. In addition, 65.13 g of tetraethoxysilane (produced by Sigma-Aldrich Co. LLC.) was added as a silica source and mixing was performed for 70 minutes at room temperature using the magnetic stirrer to yield an aqueous sol B for seed crystal preparation.

<Preparation of zeolite seed crystals B>

The aqueous sol B for seed crystals was added into a stainless steel pressure vessel including a fluororesin inner cylinder, and then a reaction (hydrothermal synthesis) was carried out for 48 hours in a 130°C hot-air dryer. Next, solid-liquid separation of the resultant reaction liquid was performed for 5 minutes by centrifugal separation in a centrifugal separator (4000 rpm), and solid content was collected. The collected solid content was dried for 12 hours in an 80°C thermostatic dryer, and then the dried solid was ground in a mortar to yield zeolite seed crystals B. It was confirmed that the resultant zeolite seed crystals B were an MFI-type zeolite by X-ray diffraction measurement. The zeolite seed crystals B had an average particle diameter of 330 nm.

(Example 3)

A separation membrane was prepared and evaluated in the same way as in Example 1 with the exception that zeolite seed crystals C prepared as follows were used instead of the zeolite seed crystals A. The results are shown in Table 1. It was confirmed that the porous separation layer of the separation membrane was an MFI-type zeolite as a result of X-ray diffraction measurement of the porous separation layer.

<Preparation of zeolite seed crystals C>

An aqueous sol B for seed crystals was prepared in the same way as in Example 2.
The aqueous sol B for seed crystals was added into a stainless steel pressure vessel including a fluororesin inner cylinder, and then a reaction (hydrothermal synthesis) was carried out for 72 hours in a 140°C hot-air dryer. Next, solid-liquid separation of the resultant reaction liquid was performed for 5 minutes by centrifugal separation in a centrifugal separator (4000 rpm), and solid content was collected. The collected solid content was dried for 12 hours in an 80°C thermostatic dryer, and then the dried solid was ground in a mortar to yield zeolite seed crystals C. It was confirmed that the resultant zeolite seed crystals C were an MFI-type zeolite by X-ray diffraction measurement. The zeolite seed crystals C had an average particle diameter of 470 nm.

(Example 4)

A separation membrane was prepared and evaluated in the same way as in Example 2 with the exception that in the membrane separation test, a mixed liquid of n-pentane and cyclopentane (mixed liquid comprising 50 mol% of n-pentane and 50 mol% of cyclopentane) was used instead of the mixed liquid of n-pentane and isopentane, and in gas chromatography measurement of the sample (condensate), the molar ratio of n-pentane and cyclopentane was determined. The results are shown in Table 1.

(Comparative Example 1)

A separation membrane was prepared and evaluated in the same way as in Example 1 with the exception that zeolite seed crystals D prepared as follows were used instead of the zeolite seed crystals A. The results are shown in Table 1. It was confirmed that the porous separation layer of the separation membrane was an MFI-type zeolite as a result of X-ray diffraction measurement of the porous separation layer.

<Preparation of aqueous sol C for seed crystals>

A magnetic stirrer was used to mix 25.35 g of tetrapropylammonium bromide (produced by Wako Pure Chemical Industries, Ltd.) as a structure directing agent and 223.55 g of ultrapure water. In addition, 3.21 g of sodium hydroxide (produced by Wako Pure Chemical Industries, Ltd.) and 46.70 g of a colloidal silica (AS-40 produced by Sigma-Aldrich Co. LLC.) dispersion liquid of 40 mass% in concentration (18.68 g in terms of silica as silica source) were added. Mixing was performed for 23 hours at room temperature using the magnetic stirrer until uniform mixing was confirmed. Thereafter, 1.19 g of ammonium fluoride (produced by Wako Pure Chemical Industries, Ltd.) was added and mixing was performed for 1 hour at room temperature to yield an aqueous sol C for seed crystal preparation.

<Preparation of zeolite seed crystals D>

The aqueous sol C for seed crystals was added into a stainless steel pressure vessel including a fluororesin inner cylinder, and then a reaction (hydrothermal synthesis) was carried out for 30 hours in a 140°C hot-air dryer. Next, solid-liquid separation of the resultant reaction liquid was performed for 5 minutes by centrifugal separation in a centrifugal separator (4000 rpm), and solid content was collected. The collected solid content was dried for 12 hours in an 80°C thermostatic dryer, and then the dried solid was ground in a mortar to yield zeolite seed crystals D. It was confirmed that the resultant zeolite seed crystals D were an MFI-type zeolite by X-ray diffraction measurement. The zeolite seed crystals D had an average particle diameter of 1140 nm.

(Comparative Example 2)

A separation membrane was prepared and evaluated in the same way as in Comparative Example 1 with the exception that in the membrane separation test, a mixed liquid of n-pentane and cyclopentane (mixed liquid comprising 50 mol% of n-pentane and 50 mol% of cyclopentane) was used instead of the mixed liquid of n-pentane and isopentane, and in the gas chromatography measurement of the sample (condensate), the molar ratio of n-pentane and cyclopentane was determined. The results are shown in Table 1.

Table 1

| | | Example 1 | Example 2 | Example 3 | Example 4 | Comparative Example 1 | Comparative Example 2 |
|---|---|---|---|---|---|---|---|
| Seed crystals | Average particle diameter [nm] | 130 | 330 | 470 | 330 | 1140 | 1140 |
| Porous support | Average pore diameter [$\mu$m] | 1.4 | 1.4 | 1.4 | 1.4 | 1.4 | 1.4 |
| Average particle diameter/ Average pore diameter [-] | | 0.09 | 0.24 | 0.34 | 0.24 | 0.81 | 0.81 |
| Porous separation layer | Thickness [$\mu$m] | 20 | 11 | 13 | 11 | 16 | 16 |
| | c-Axis/(a-Axis + b-Axis) [-] | 5.28 | 5.76 | 6.51 | 5.76 | 2.16 | 2.16 |
| | $\sum(10x)/(101)$ [-] | 1.64 | 1.64 | 1.17 | 1.64 | 1.06 | 1.06 |
| Separation factor $\alpha$ [-] | | 83.50 | 66.20 | 45.30 | 14.41 | 73.50 | 12.31 |
| Permeation flux F [kg/m²·h] | | 2.30 | 4.70 | 2.99 | 1.13 | 1.14 | 0.64 |
| F×$\alpha$[kg/m²·h] | | 192.05 | 311.14 | 135.24 | 16.28 | 83.79 | 7.88 |

It can be seen from Table 1 that in cases in which separation membranes of the examples were used, compared to cases in which separation membranes of the comparative examples were used, it was possible to efficiently separate n-pentane from a mixed liquid containing n-pentane and isopentane or efficiently separate n-pentane from a mixed liquid containing n-pentane and cyclohexane.

INDUSTRIAL APPLICABILITY

According to this disclosure, it is possible to provide a separation membrane that when used in membrane separation of a linear hydrocarbon and a branched hydrocarbon and/or cyclic hydrocarbon of equivalent carbon number to the linear hydrocarbon, can efficiently separate the linear hydrocarbon and the branched hydrocarbon and/or cyclic hydrocarbon.

REFERENCE SIGNS LIST

| 1 | test apparatus |
|---|---|
| 2 | feedstock tank |
| 3 | liquid feed pump |
| 4 | first heat exchanger |
| 5 | separator |
| 6 | cold trap |
| 7 | second heat exchanger |
| 8 | back pressure valve |
| 9 | pressure gauge |
| 10, 14 | three-way valve |
| 11 | vacuum pump |
| 12 | sampling valve |
| 13 | sampling cold trap |

**Claims**

1. A separation membrane for use in membrane separation of a hydrocarbon mixture containing a linear hydrocarbon and either or both of a branched hydrocarbon and a cyclic hydrocarbon of equivalent carbon number to the linear hydrocarbon, the separation membrane comprising:

   a porous support; and a porous separation layer disposed on the porous support and containing an MFI-type zeolite, wherein
   in an X-ray diffraction pattern obtained through X-ray diffraction measurement of the porous separation layer: a total intensity of diffraction peaks attributed to MFI-type zeolite (001) planes, (002) planes, (004) planes, (101) planes, (102) planes, (103) planes, (104) planes, (105) planes, (202) planes, and (303) planes is at least 3 times the sum of a total intensity of diffraction peaks attributed to MFI-type zeolite (100) planes, (200) planes, (400) planes, (301) planes, and (501) planes and a total intensity of diffraction peaks attributed to MFI-type zeolite (010) planes, (020) planes, (040) planes, and (051) planes; and a total intensity of diffraction peaks attributed to MFI-type zeolite (101) planes, (102) planes, (103) planes, (104) planes, and (105) planes is less than 3 times an intensity of a diffraction peak attributed to MFI-type zeolite (101) planes.

2. The separation membrane according to claim 1, wherein
   the hydrocarbon mixture is a mixture containing, as main components, a linear hydrocarbon having a carbon number of 4 and either or both of a branched hydrocarbon having a carbon number of 4 and a cyclic hydrocarbon having a carbon number of 4, or a mixture containing, as main components, a linear hydrocarbon having a carbon number of 5 and either or both of a branched hydrocarbon having a carbon number of 5 and a cyclic hydrocarbon having a carbon number of 5.

3. A method of producing a separation membrane, for use in producing the separation membrane according to claim 1 or 2, comprising
   immersing a porous support having one or more zeolite seed crystals adhered thereto in an aqueous sol containing a silica source and a structure directing agent, and synthesizing a zeolite including an MFI-type zeolite by hydrothermal synthesis to form a porous separation layer on the porous support, wherein
   the zeolite seed crystals have an average particle diameter of at least 50 nm and no greater than 700 nm, and
   a ratio of the average particle diameter of the zeolite seed crystals relative to an average pore diameter of the porous support is at least 0.01 and no greater than 0.7.

# FIG. 1

## INTERNATIONAL SEARCH REPORT

| | International application No. |
|---|---|
| | PCT/JP2016/000375 |

**A. CLASSIFICATION OF SUBJECT MATTER**
*B01D71/02*(2006.01)i, *B01D69/10*(2006.01)i, *B01D69/12*(2006.01)i, *C01B39/36* (2006.01)i

According to International Patent Classification (IPC) or to both national classification and IPC

**B. FIELDS SEARCHED**

Minimum documentation searched (classification system followed by classification symbols)
B01D71/02, B01D69/10, B01D69/12, C01B39/36

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2007-517648 A (BASF AG.),<br>05 July 2007 (05.07.2007),<br>claims 1 to 2, 5 to 6; paragraphs [0023],<br>[0027], [0068]<br>& US 2007/0137485 A1<br>claims 1 to 2, 5 to 6; paragraphs [0022],<br>[0030], [0080]<br>& WO 2005/068057 A1 & EP 1706197 B1<br>& DE 102004001974 A1 & CN 1909954 A | 1-3 |
| Y | JP 2010-247150 A (Mitsubishi Chemical Corp.),<br>04 November 2010 (04.11.2010),<br>paragraph [0034]<br>& US 2012/0074065 A1<br>paragraph [0076]<br>& WO 2010/110274 A1 & EP 2412427 A1<br>& CN 102361683 A | 1-3 |

| ☒ | Further documents are listed in the continuation of Box C. | ☐ | See patent family annex. |
|---|---|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 April 2016 (06.04.16) | 19 April 2016 (19.04.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.

PCT/JP2016/000375

C (Continuation). DOCUMENTS CONSIDERED TO BE RELEVANT

| Category* | Citation of document, with indication, where appropriate, of the relevant passages | Relevant to claim No. |
|---|---|---|
| A | WO 2007/058388 A1  (Nippon Glass Co., Ltd.), 24 May 2007 (24.05.2007), entire text & US 2008/0217240 A1 whole documents & CA 2629756 A1 | 1-3 |
| P,X | JP 2015-160186 A  (Nippon Zeon Co., Ltd.), 07 September 2015 (07.09.2015), paragraphs [0053] to [0063] (Family: none) | 1-3 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007517648 A